# EUROPEAN PATENT APPLICATION

(11) **EP 2 463 379 A1**
(43) Date of publication of application: **13.06.2012**
(21) Application number: 10804072.6
(22) Date of filing: 15.07.2010
(51) Int. Cl.: C12Q 1/02, C12M 1/34, G01N 21/17, G01N 33/483, G06T 1/00

(54) **TECHNIQUE FOR DETERMINING MATURITY OF CELL MASS, AND IMAGE PROCESSING PROGRAM AND IMAGE PROCESSING DEVICE WHICH USE THE TECHNIQUE, AND METHOD FOR PRODUCING CELL MASS**

(30) Priority: 31.07.2009 JP 2009179824
(71) Applicant: Nikon Corporation, Tokyo 100-8331 (JP)
(72) Inventor: MIMURA, Masafumi, Tokyo 100-8331 (JP); ITO, Kei, Tokyo 100-8331 (JP); YANO, Kazuhiro, Tokyo 100-8331 (JP)
(74) Representative: HOFFMANN EITLE
(86) International application number: PCT/JP2010/004597
(87) International publication number: WO 2011/013319

(57) **Abstract**

An image processing program (GP) is configured to comprise a step (A10) for obtaining time lapse images in which a cell aggregation is imaged over a predetermined time interval by an imaging device, a step (A20) for sequentially calculating a feature value relating to multi-layering of the cell aggregation from the obtained time lapse images, a step (A30) for deriving time lapse changes in the calculated feature value relating to the multi-layering, and a step (A40) for outputting the derived time lapse changes in the multi-layering feature values.

## Description

### TECHNICAL FIELD

The present invention relates to a maturity analysis technique for determining the degree of maturity of a cell aggregation, from time lapse images obtained during cell observation.

### TECHNICAL BACKGROUND

A cell culture microscopy can be cited as an example of a device for observing a cell while the cell is being cultured. A cell culture microscopy is provided with a cell culture device for forming an environment suitable for culturing a cell, and a microscope observation system for microscopic observation of a cell in a cell culture container. The cell culture microscopy is configured so that changes, divisions, and other cell activities can be observed while the living cell is cultured (see Patent Document 1, for example). In the live cell culturing stage, a cell aggregation is formed by the progression of cellular division and integration between cells. In the initial stage of a flat cell culture, the cells spread out in the horizontal direction through the cell culture medium in a single-layered state, but then as cellular division becomes more active and the cell aggregation grows, the cells also spread out in the up-down direction so as to form bubbles, and so-called "multi-layering" progresses.

In a conventional cell observation technique using a cell culture microscopy, the degree of maturity of a cell aggregation is determined visually, where a determination is made according to visual observation of a microscope observation image, and/or by a reagent determination, where a reagent is administered, the degree of activity of cell division or other parameter is detected, and a determination is made according to the degree of maturity.

### PRIOR ARTS LIST

### PATENT DOCUMENT

Patent Document 1: Japanese Laid-open Patent Publication No. 2004-229619 (A)

### SUMMARY OF THE INVENTION

### PROBLEMS TO BE SOLVED BY THE INVENTION

However, the conventional technique for visual determination has been problematic in that an expert having a certain amount of experience must make observations over time, and in that qualitatively evaluating and deciding the degree of maturity of the cell aggregation is difficult, and it is impossible to obtain sufficient reproducibility and/or reliability. The technique for a reagent judgment has been further problematic in that administering the reagent has a major chemical and physical effect on the cells, and in that there are major constraints in using cultured cells.

The present invention was developed in view of such problems, it being an object of the present invention to provide means by which the degree of maturity of a cell aggregation can be determined from time lapse images taken by an imaging device without the cells being damaged due to the administration of a reagent.

### MEANS TO SOLVE THE PROBLEMS

According to a first aspect of the present invention, provided is a technique for determining the maturity of a cell aggregation, comprising the steps of obtaining time lapse images in which images of a cell aggregation are taken by an imaging device over a predetermined time interval; sequentially calculating a feature value relating to multi-layering of the cell aggregation from the obtained time lapse images, and deriving time lapse changes of the calculated feature value relating to the multi-layering; and determining the degree of maturity of the cell aggregation on the basis of the time lapse changes of the feature value relating to the multi-layering.

According to a second aspect of the present invention, provided is an image processing program that can be read out by a computer, the image processing program being adapted for causing the computer to function as an image processing device for obtaining an image taken by an imaging device and performing image processing, comprising the steps of obtaining time lapse images in which images of a cell aggregation are taken by an imaging device over a predetermined time interval; sequentially calculating a feature value relating to multi-layering of the cell aggregation from the obtained time lapse images; deriving time lapse changes of the calculated feature value relating to the multi-layering; and outputting information on the derived time lapse changes in the feature value relating to the multi-layering.

According to a third aspect of the present invention, an image processing device is configured to be provided with an image analysis unit for obtaining time lapse images in which images of a cell aggregation are taken by an imaging device over a predetermined time interval and analyzing the images, and an output unit for outputting a result of the analysis performed by the image analysis unit. The image processing device is configured such that the image analysis unit sequentially calculates a feature value relating to multi-layering of the cell aggregation from the time lapse images, and derives time lapse changes in the calculated feature value relating to the multi-layering; and the output unit outputs information on the time lapse changes in the feature value relating to the multi-layering as derived by the image analysis unit.

In a preferred aspect of the present invention described above, the feature value relating to the multi-layering is a statistic based on the degree of similarity (for example, a correlation value, difference value, multiplication value, or the like) of a region at a position with the highest degree of matching calculated for a first image at a time t and a second image at a time t+1 in the time lapse images, using the luminance distribution of a local region of the cell aggregation in the first image as a template, and performing block matching for the luminance distribution of a nearby part including a corresponding position of the cell aggregation in the second image. In another preferred aspect, the feature value relating to the multi-layering is a statistic that is based on a pixel value in the neighborhood of a contour of the cell aggregation. Alternatively, in another preferred aspect, the feature value relating to the multi-layering is a statistic that is based on a shape of the contour of the cell aggregation.

The image processing program or image processing device of the present invention is preferably configured so as to determine the degree of maturity of the cell aggregation on the basis of the information on the time lapse changes of the feature value relating to the multi-layering, and to output a result of the determination. In a preferred configuration, in a case in which the time lapse images include a plurality of cell aggregations, the degree of maturity is determined for each of the cell aggregations, a distinction is made between matured cell aggregations and immature cell aggregations, and a result of the distinction is outputted.

### ADVANTAGEOUS EFFECTS OF THE INVENTION

In the technique for determining the maturity of a cell aggregation, the image processing program, and the image processing device of the present invention, the feature value relating to the multi-layering of the cell aggregation is sequentially calculated from the time lapse images in which images of the cell aggregation are taken over a predetermined time interval by an imaging device, the time lapse changes are derived, and the degree of maturity of the cell aggregation is decided on the basis of the time lapse changes in the feature value relating to the multi-layering. Therefore, according to the present invention, there can be provided means by which the degree of maturity of a cell aggregation can be decided from time lapse images taken by an imaging device without the cells being damaged due to the administration of a reagent.

### BRIEF DESCRIPTION OF THE DRAWINGS

FIG. 1 : A flow chart illustrating an example of an image processing program GP1 of a first structural mode;
FIG. 2 : A diagram providing a rough structural view of a cell culture observation system illustrated as an example of the application of the present invention;
FIG. 3 : A block diagram of the aforementioned cell culture observation system;
FIG. 4 : A block diagram illustrating an overview of an example of a configuration of an image processing device;
FIG. 5 : A sub-flow chart of the image processing programs of the first and second models;
FIG. 6A-6B : FIG. 6A shows a first image of a cell aggregation and FIG. 6B shows a second image of the cell aggregation, taken over a predetermined time interval;
FIG. 7 : A schematic illustrating an example of the status of a cell aggregation that has been segmented and labeled;
FIG. 8A-8B : FIG. 8A shows an example of the configuration of a local region that is set in the first image, and FIG. 8B shows an explanatory view for describing the status in which block matching is executed for a nearby part that includes the corresponding position in the second image;
FIG. 9A-9B : FIG. 9A shows an explanatory view illustrating an example of the size of the local region relative to the cell aggregation, and FIG. 9B shows an example of the configuration for displaying the degree of multi-layering calculated by the image analysis unit;
FIG. 10 : An example of the output of the time lapse information of the degree of multi-layering, graphically showing the temporal changes in the sum of the degrees of multi-layering;
FIG. 11 _{:} A flow chart illustrating an example of an image processing program GP2 of a second structural mode;
FIG. 12 _{:} An example, of the output of the time lapse information on multi-layering occupancy, graphically showing the temporal changes in the occupancy associated with multi-layering;
FIG. 13A-13B : FIG 13A shows an explanatory view providing a schematic illustration of observation images of a cell aggregation at the initial stage of cell culturing and FIG. 13B shows an explanatory view providing a schematic illustration of observation images of a cell aggregation at the mature state in which the multi-layered region has spread out to all regions.
FIG. 14 : A flow chart illustrating an example of an image processing program GP3 of a third structural mode;
FIG. 15 _{:} An example of the output of time lapse information on a luminance statistic, graphically showing the temporal changes of the sum of pixel values in the neighborhood of the contours of the cell aggregation;
FIG. 16 _{:} A flow chart illustrating an example of an image processing program GP4 of a fourth structural mode;
FIG. 17 : An example of the output of the time lapse information on the contour shape statistic, graphically showing the temporal changes in the degree of complexity of the contours of the cell aggregation; and
FIG. 18 : A flow chart illustrating a method for producing a cell aggregation.

### DESCRIPTION OF THE EMBODIMENTS

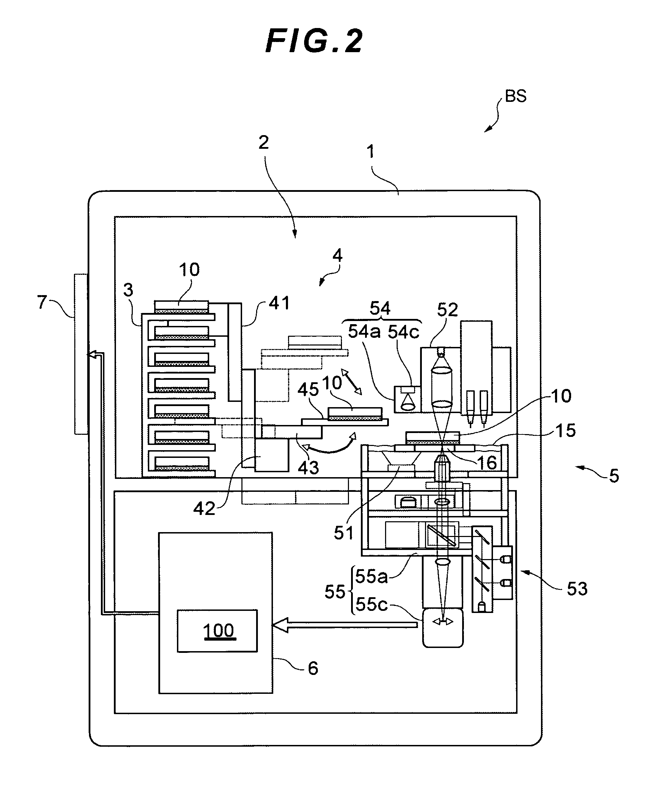

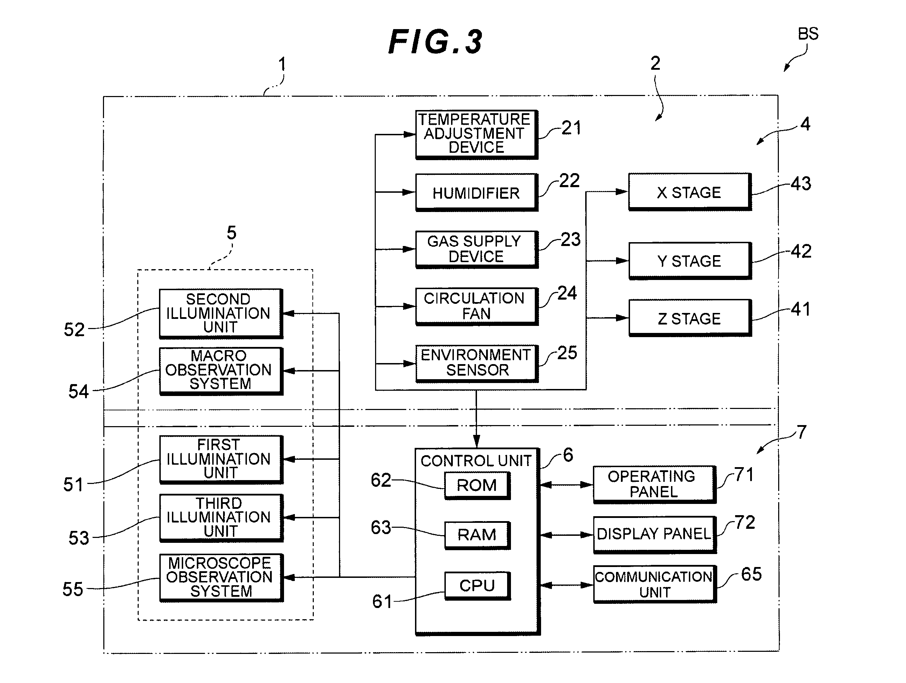

Embodiments of the present invention will be described herein after with reference to the accompanying drawings. As an example of a system in which the image processing device of the present invention has been applied, FIGS. 2 and 3 illustrate a rough structural view and a block diagram of a cell culture observation system. First, a description of the overall configuration of a cell culture observation system BS will be summarized.

The cell culture observation system BS is broadly constituted of a cell culture chamber 2 provided to a top part of a chassis 1; a stocker 3 for accommodating and retaining a plurality of cell culture containers 10; an observation unit 5 for observing samples in the cell culture containers 10; a conveyance unit 4 for conveying the cell culture containers 10; a control unit 6 for controlling the operation of the system; an operating board 7 provided with an image display device; and other components.

The cell culture chamber 2 is a compartment for forming a cell culture environment, and the cell culture chamber 2 is additionally provided with such components as a temperature adjustment device 21; a humidifier 22; a gas supply device 23 for supplying CO₂ gas, N₂ gas, or other gas; a circulation fan 24; and an environment sensor 25 for detecting the temperature, humidity, and other features of the cell culture chamber 2. The stocker 3 is formed in a shelf shape partitioned in the front-rear and up-down directions, a specific number being set for each shelf. The cell culture container 10 is appropriately selected according to the type or purpose of the cell to be cultured; cell samples are injected together with a liquid cell culture medium and retained in, for example, dish-type cell culture containers. A code number is assigned to each of the cell culture containers 10, which are associated with a designated number and accommodated in the stocker 3. The conveyance unit 4 comprises such components as a Z stage 41 capable of moving up and down, a Y stage 42 capable of moving forward and backward, and an X stage 43 capable of moving left and right, these stages being provided within the cell culture chamber 2. A support arm 45 for lifting and supporting a cell culture container 10 is provided toward the distal end of the X stage 43.

The observation unit 5 is constituted of such components as a first illumination unit 51 for illuminating a sample from a lower side of a sample stage 15; a second illumination unit 52 for illuminating the sample along the optical axis of a microscope observation system 55 from above the sample stage 15; a third illumination unit 53 for illuminating the sample from below; a macro observation system 54 for macro observation of the sample; a microscope observation system 55 for micro observation of the sample; and an image processing device 100. A transparent window part 16 is provided to the sample stage 15, in the region thereof observed by the microscope observation system 55.

The macro observation system 54 is configured to have an observation optical system 54a and a CCD camera or other imaging device 54c for taking an image of a sample that is imaged by the observation optical system. An overall observation image (macro image) is obtained from above the cell culture container 10, which is backlit by the first illumination unit 51. The microscope observation system 55 is configured to have an observation optical system 55a comprising an objective lens, a middle zooming lens, a fluorescence filter, and other components; and a cooled CCD camera or other imaging device 55c for taking an image of the sample imaged by the observation optical system 55a. The objective lenses and middle zooming lenses are provided in pluralities, and are configured such that the desired magnification for observation can be set by altering the combination of lenses. The microscope observation system 55 obtains a transmittance image of a cell illuminated by the second illumination unit 52; a reflection image of a cell illuminated by the third illumination unit 53; a fluorescence image of a cell illuminated by the third illumination unit 53, and other microscope observation images (micro images) in which the cell inside the cell culture container 10 is microscopically observed.

Images are taken by the imaging device 54c of the macro observation system 54 and the imaging device 55c of the microscope observation system 55, the image processing device 100 processing the signals inputted from these imaging device; and generating an image of the overall observation image, the microscope observation image, or the like. The image processing device 100 applies image analysis to (the image data of) the observation images, generates a time lapse image, predicts a movement direction of a cell, analyzes the motion state of the cell, analyzes the state of change of a cell aggregation toward becoming multi-layered, and performs other processing. The image processing device 100 will be described in detail hereinafter.

The control unit 6 has a CPU 61 for executing professes; a ROM 62 in which a control program, control data, or the like for the cell culture observation system BS are set and stored; and a RAM 63 for temporarily storing observation conditions, image data, and the like, which comprises a hard drive, DVD, or other auxiliary storage device; and other components; and controls operation of the cell culture observation system BS. Therefore, as illustrated in FIG. 3, the respective constituent instruments of the cell culture chamber 2, the conveyance unit 4, the observation unit 5, and the operating board 7 are connected to the control unlit 6. Environment conditions of the cell culture chamber 2, an observation schedule, and observation classifications, observation positions, observation magnifications, and other information for the observation unit 5 are set and stored in the RAM 63, in accordance with the observation program. The RAM 63 is also provided with an image data memory region for recording image data taken by the observation unit 5. Index data, which include a code number of the cell culture container 10, an image-capture date and time, and other information, are recorded in association with image data.

The operating board 7 is provided with an operating panel 71 to which a keyboard, switch, or other input/output instrument is provided; and with a display panel 72 for displaying an operating screen, an observation image, analysis results, or the like. On the operating panel 71, the observation program is set, the conditions are selected, and an operational instruction or the like is inputted. A communication unit 65 is configured to conform to a wired or wireless communication standard, permitting data to be sent from and received by a computer or the like that is externally connected to the communication unit 65.

In the cell culture observation system BS thus generally configured, the CPU 61 controls the operation of each of the components and automatically photographs the sample in the cell culture container 10, in accordance with the observation program that has been set in the operating board 7. When the observation program is started, the CPU 61 controls the operation of the temperature adjustment device 21, the humidifier 22, and the like, on the basis of the environment conditions stored in the RAM 63. The observation conditions stored in the RAM 63 are read in; the X, Y, and Z stages 43, 42, 41 are operated on the basis of the observation schedule; the cell culture container 10 that is to be observed is conveyed from the stocker 3 to the sample stage 15; and the observation by the observation unit 5 is initiated. In a case where, for example, the observation that has been set in the observation program is micro observation of a cell, the corresponding cell culture container 10 is positioned onto the optical axis of the microscopic observation system 55, the light source of the second illumination unit 52 or the third illumination unit 53 is activated, and the imaging device 55c is made to take a microscopic observation image.

The cell culture observation system BS configured as described above has a function whereby the image processing device 100 obtains time lapse images taken by an imaging device (54c, 55c) over a predetermined time interval, analyzes the obtained time lapse images, and outputs information that is useful for quantitatively evaluating and deciding the degree of maturity of the cell aggregation. Such a function can be used appropriately to analyze, for example, iPS cells, ES cells, or the like.

Feature values relating to the multi-layering of a cell aggregation (herein after referred to "multi-layering feature values" for convenience) are sequentially calculated from the obtained time lapse images, the time lapse changes in the calculated multi-layering feature values are derived, and the degree of maturity of the cell aggregation is decided on the basis of the time lapse changes in the multi-layering feature values.

In a flat cell culture, where the cell culture cells spread out in a flat manner within the cell culture medium, mnlti-layering begins during the stage by which iPS cells, ES cells, or other cells grow into a cell aggregation in a quasi-societal manner; and the multi-layering spreads out into the entire region of the cells mass as maturation proceeds. Therefore, the multi-layering feature values of the cell aggregation are calculated at each of the observation images of the time lapse images or between the images, and the temporal changes thereof are derived, whereby the degree of maturity of the cell aggregation (the state of maturity) can be decided.

The present invention proposes, as multi-layering feature values serving as the basis for deciding the degree of maturity, (I) a technique for using a statistic based on the degree of similarity by block matching of a local region between the observation images, (II) a technique for using statistics based on the pixel values in the neighborhood of the contours of the cell aggregation, and (III) a technique for using a statistic based on the contour shape of the cell aggregation. The following provides a respectively divided and ordered description for such techniques for deciding the degree of maturity of a cell aggregation using the multi-layering feature values.

(I) Technique for using the degree of similarity by block matching of a local region between observation images
This technique addresses an observation image at a time t (a first image) and an observation images at a time t+1 (a second image) in time lapse images (at times t=0, 1, 2, 3, ..., T, T+1, ...) taken in a predetermined time interval, and uses the luminance distribution of the local region of the cell aggregation in the first image as a template for block matching the luminance distribution for an nearby part including a corresponding position of the cell aggregation in the second image. The calculated degree of similarity of the region of the position having the highest degree of matching (the region of the position with the least change in luminance distribution within the region) serves as a representative degree of similarity of the region of the relevant position, and a statistic based on the representative degree of similarity serves as the multi-layering feature value.

This technique makes use of the fact that the images of a site of a single-layered region, in which the cells have not become multi-layered, and of a multi-layered site have the following features. Although a cell aggregation is a plurality of aggregated cells, the size of cells and the boundaries therebetween in a single-layered cell aggregation where the cells aggregate and spread out in the horizontal direction in a simple manner can be observed even when some movement and/or rotation has occurred in individual cells between images of adjacent points in time, and the structure thereof is regarded as being preserves. On the other hand, in a case where the cells becomes multi-layered, a change occurs such that division and/or movement takes place in the up/down direction in the interior of the cell aggregation and bubbles are formed; therefore, the spatial structure and brightness of the images change dramatically.

Thus, since the changes in the interior of the cell aggregation in a single-layered region are primarily the spatial movement, the degree of matching when block matching is performed in the periphery including the corresponding position of two images is accordingly higher; however, in a multi-layered region, the changes in the interior of the cell aggregation not only relate to the spatial movement but are structural i11 nature, and the degree of matching accordingly decreases even when the periphery is searched. A correlation value, a difference value, a multiplication value, or another parameter can be used as an index for the degree of similarity. For example, in a case where a correlation value is used, the representative degree of similarity will be high in a single-layered region, but low in a multi-layered region; and the state of multi-layering can be decided depending on the magnitude of the representative degree of similarity.

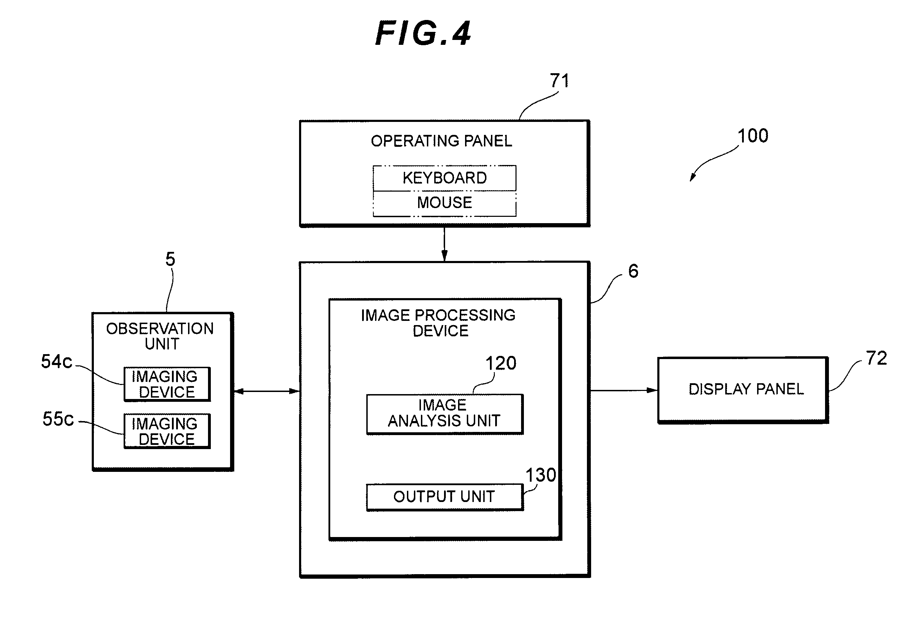

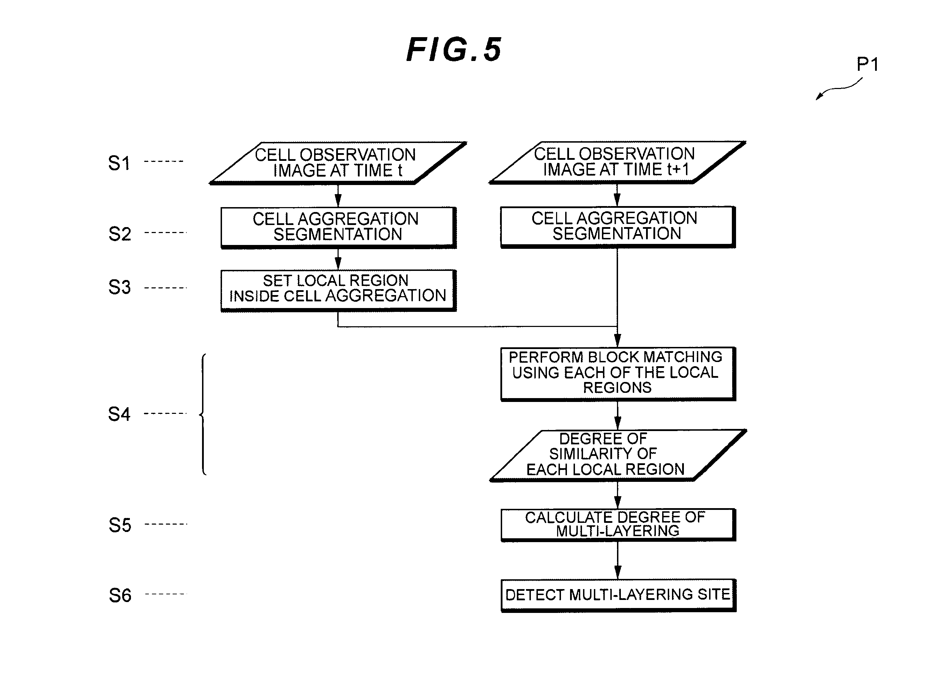

Accordingly, moving the local region in the first image and performing block matching for the entire region of the image (or, a designated region, in a case in which an analysis range is designated using a mouse or the like) makes it possible to ascertain the state of change of each of the parts of the cell aggregation toward becoming multi-layered at each time (time t = 1, 2, 3 , ...). Then, deriving the time lapse changes thereof makes it possible to sequentially decide the state of change of each of the parts of the cell aggregation and of the entire cell aggregation toward becoming multi-layered. Block matching of the local region between images is executed in the image processing device 100. FIG. 4 illustrates a block diagram of the image processing device 100, and FIG. 5 illustrates a flow chart of a portion P1 (a sub-flow chart) for performing a detection process for a multi-layered site in the image processing programs GP1, GP2 of this technique.

The image processing device 100 is configured to be provided with an image analysis unit 120 for obtaining images of a cell aggregation taken by an imaging device (55c, 54c) and analyzing the images, and an output unit 130 for outputting the analysis results from the image analysis unit 120. The analysis results from the image analysis unit 120 are outputted from the output unit 130 and displayed on the display panel 72, recorded to a storage medium, sent as a data transmission to an external unit via the communication unit 65, or the like, the analysis results being, for example, information on the time lapse changes in the multi-layering feature values, the results from determining the degree of maturity, the results from identifying a matured cell aggregation and an immature cell aggregation, or the like.

The image processing programs GP (GP1 to GP4), which are set and stored in the ROM 62, are read into the CPU 61, and processing based on the image processing programs GP is executed sequentially by the CPU 61, whereby the image processing device 100 is configured. In other words, the image processing programs GP are software serving to cause the CPU 61 (a computer), which is a hardware resource, to function as the image processing device 100.

The image analysis unit 120 runs the image processing described below on the basis of the image processing programs GP for the images of the cell aggregation, which are taken by an imaging device (for the purpose of description, reference is made here to the imaging device 55c of the micro system) and recorded in the RAM 63. When the second image has been taken by the imaging device 55c, the state of change of the cell aggregation toward becoming mufti-layered at the current point in time may also be subjected to image processing and outputted in real time, from the first image, which is recorded in the RAM 63, and the second image, which has been obtained anew.

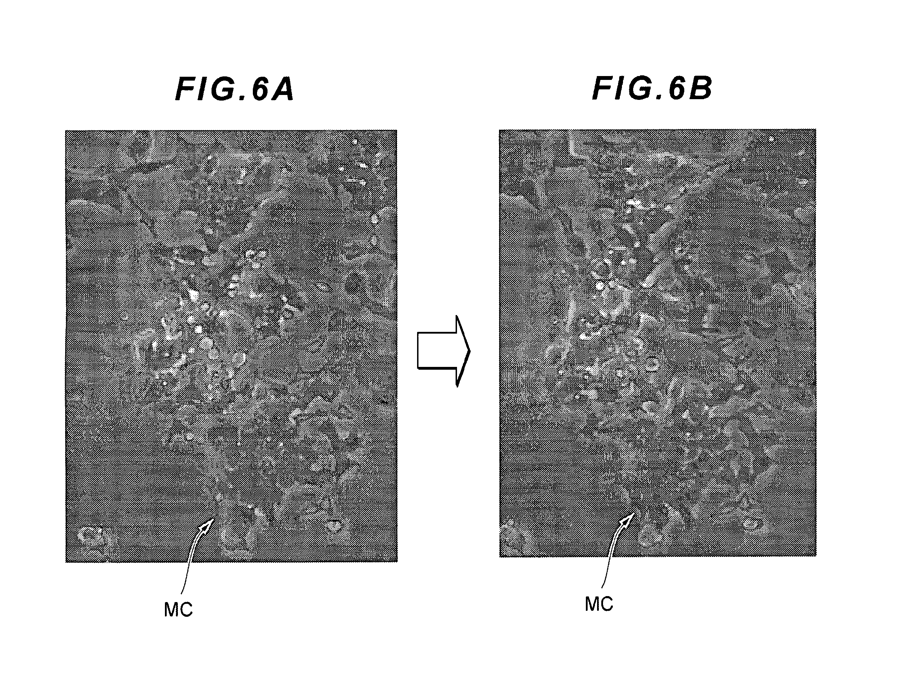

The image analysis unit 120 first obtains, in step S1, a first image at a time t that is stored in the RAM 63 (for example, the cell observation image illustrated in FIG. 6A) and a cell observation image at a subsequent time t+1 at a predetermined time interval (for example, the cell observation image illustrated in FIG. 6B), and, in step S2, segments the cell aggregations MC by the Level Set method and variance filtering for each of the images.

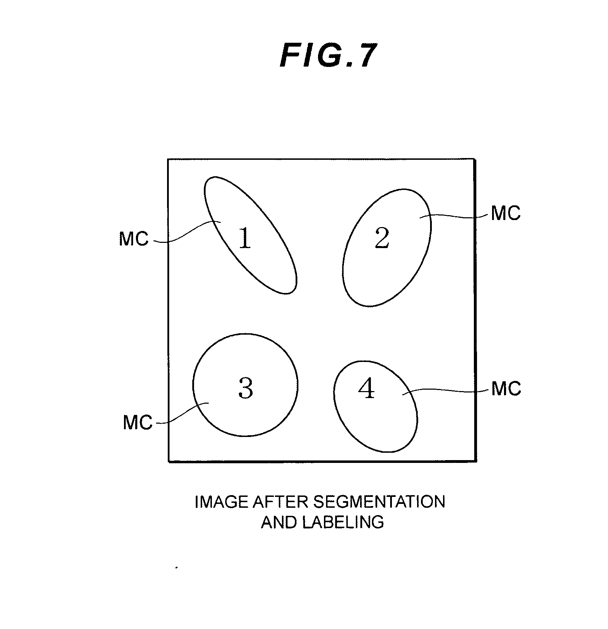

At such a time, in a case where the image includes a plurality of segmented cell aggregations MC, as illustrated in FIG. 7, each of the cell aggregations MC is labeled and correspondences are made for the cell aggregations between the first image and the second image. For example, as regards cell aggregations MC labeled "1," "2," "3 ..." in each of the images, a correspondence is made such that labels overlapped by an image relate to the same cell aggregation. The aforementioned predetermined time interval is appropriately selected in accordance with the type and/or activity status of the cells that are 10 be observed, but the linages are selected over an interval of time on the order of ten minutes 10 one hour in a case where the ceils are very active, or on the order of 30 minutes 10 two hours in a case where the cells are not very active.

Next, the cell aggregations MC are aligned in order to reduce the effect arising from the cell aggregations moving from the first image to the second image (not shown). The position of the center of gravity of the cell aggregation, the vertex positions of the rectangular contour, or the like can be used as a standard for alignment; the angle of the cell aggregations can be accounted for so as to maximize the correlation of the moment of the shape (minimize the difference), whereby the effects of rotation can be reduce. The alignment may be done at the position and angle at which the difference of the contour shapes and/or luminance distribution of each of the cell aggregations reaches a minimum (the correlation value reaches a maximum).

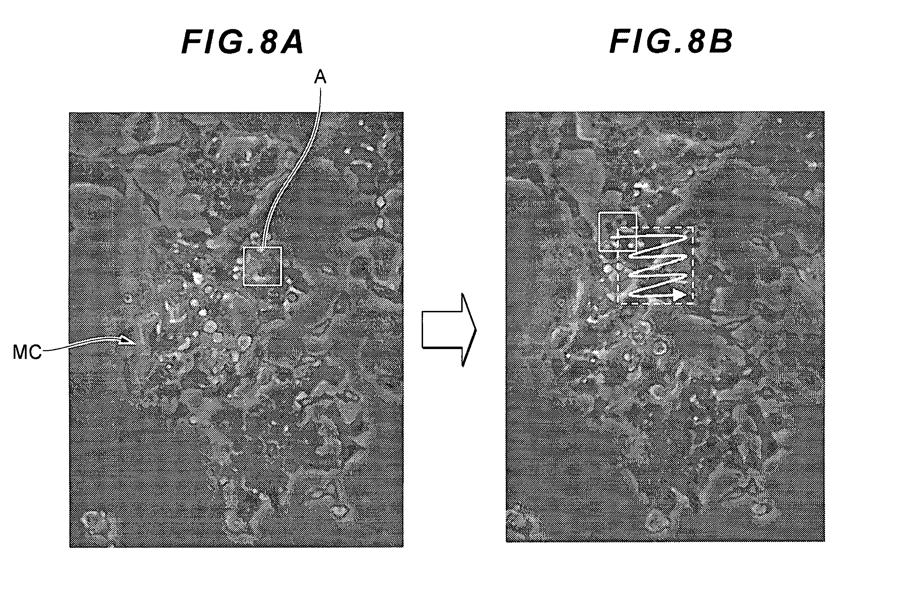

In step S3, a local region A centered on the pixels forming the first image is set in the cell aggregation MC of the image. The "local region" A, which in FIG. 8A is illustrated as enclosed by a white-bordered frame, is set sufficiently smaller than the size of the cell aggregation, and is set to, for example, approximately 5 x 5 to 15 x 15 pixels (which is the approximate size of several cells). The setting of the position of the local region can be configured so as to be an automatic setting where the contour edges of the cell aggregations that have been segmented serve as starting points; in addition, in a case in which, for example, an operator uses a mouse or the like to designate an analysis range and executes analysis for a specific portion of a cell aggregation (a portion of interest), the configuration may be such that the edges or middle of the designated analysis range are set as a starting point.

Block matching is performed in step S4 for the local region set in this manner. In block matching, the luminance distribution of the local region A set as in FIG. 8A in the first image is used as a standard; a scan is made of the luminance distribution of the local region A relative to the periphery that includes the region of the corresponding position in the second image, as illustrated in FIG. 8B; the degree of similarity at each position is calculated; and a search is made for the position that has the highest degree of matching.

A luminance distribution correlation value, difference, multiplication value, or other value can be used as an index for evaluating the degree of similarity. For example, in a case where the correlation value is used, a search is made for the position having the greatest correlation value (approaching 1), and in a case where the difference is used as the evaluation index, a search is made for the position having the smallest difference (approaching 0). Then, the degree of similarity of the position having the highest degree of matching is recorded in the RAM 63 as the representative degree of similarity of the relevant position. The description of a case where the correlation value is used as the degree of similarity continues below.

In a case in which the local region has a single-layered structure, the changes of a cell aggregation over time are primarily composed of cellular movement; therefore, performing block matching at a peripheral part that includes the corresponding position results in a high value for the correlation value of the representative degree of correlation (the correlation value is a value approaching 1). On the other hand, in a case in which the local region is a site that has become multi-layered, the changes of the cell aggregation over time involve deformation of the spatial structure as well as luminance shifts; therefore, the correlation value of the representative degree of similarity is a smaller value (as above, a value approaching 0) even when the periphery is searched.

In step S4, the local region A of the first image, serving as a comparative standard, is moved a predetermined number of pixels (a single pixel or a plurality of pixels) within the image to perform sequential block matching, and the representative degree of similarity of each of the parts is calculated for the entire region of the cell aggregation (the designated region in a case where an analysis range is designated). The representative degree of similarity of each of the parts obtained by the block matching represents the state of change of each of the parts toward becoming multi-layered, and the distribution of the representative degrees of similarity represents the status of the entire cell aggregation toward becoming multi-layered.

Herein, in a case in which a correlation value is used as the degree of similarity, the magnitude of the correlation value (the absolute value) becomes a smaller value as the multi-layering progresses from a single-layered state, and the extent of multi-layering is less readily represented. In view whereof, the image processing device 100 performs processing such that the correlation value of the representative degree of similarity is inverted in step S5 and increases (from 0 approaching 1) as the multi-layering progresses. Specifically, in a case where the correlation value is used as the degree of similarity, the processing involves subtracting the correlation value from 1, and in a case where the difference value is used as the degree of similarity, the processing involves taking the absolute value of the difference value. In the Specification, the representative degree of similarity calculated by such processing is denoted as the "degree of multi-layering." A value of -1 to +1 can be adopted as the correlation value in the computations, but a negative value (-1 to 0) reflects a case in which the luminance is inverter. Since this is of no significance in terms of the cell shape, 0 is used in a case where the calculated correlation value is a negative value, and the process of subtracting the correlation value from 1 is performed.

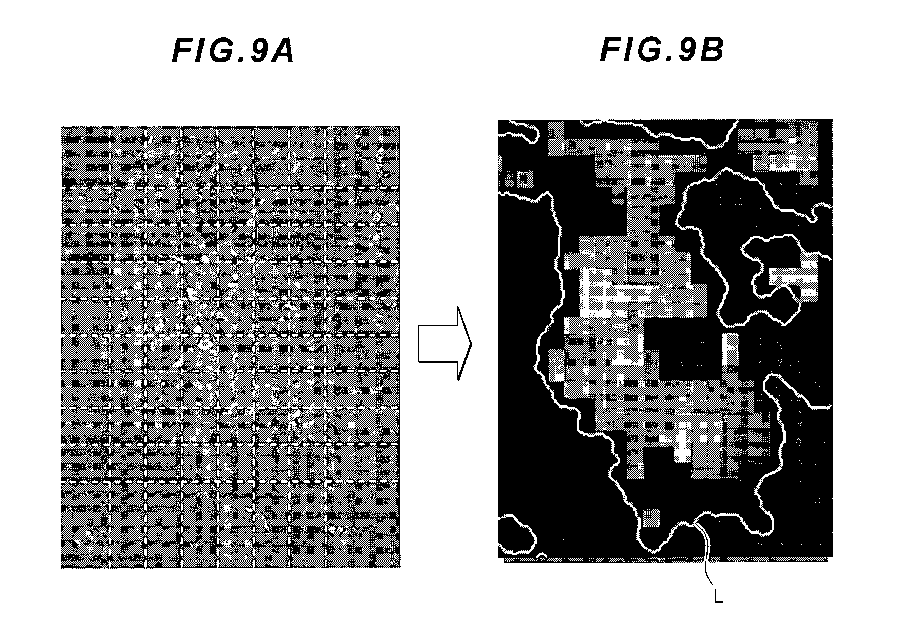

The dotted line in FIG. 9A illustrates the size of the local region, and FIG. 9B illustrates an example of the output in a case where the distribution of the degrees of multi-layering calculated by step S5 is outputted to the display panel 72 or the like as multi-layering information. In this example of output, the outer contour line L extracted in step S2 is additionally displayed in the cell aggregation of the second image, and the display is a multi-level gradation display in accordance with the magnitude of the degree of multi-layering, where a site having a low degree of multi-layering is dark and a site having a high degree of multi-layering is bright. From this image, the degree to which the multi-layering can be decided to have progressed from this image is proportional to the luminosity in the interior of a cell aggregation MC surrounded by the outer contour line L (a shade closer to white on the grey scale), and it is possible to decide the degree to which the miati-layeting has progressed on a given site of the cell aggregation.

The image processing program P1 is also provided with a function for using the degree of multi-layering calculated in step S5 to identify a site that has become multi-layered and a site that has not becomes multi-layered. Specifically, in step S6, the degree-of-multi-layering value calculated in S5 is compared against a pre-set predetermined threshold, and multi-layering is decided to have occurred in a region having a degree-of-multi-layering value at or above the threshold. The processing in step S6 is executed in accordance with a process flow of the image processing program (described below), a display request from the operator, or the like.

There may also be adopted a configuration such that the spatial change in luminance (disparity of pixel values) is calculated for the region of the position decided to have the greatest approximation by the block matching in step S4, from the luminance distribution of the region; in step S6, when the degree of multi-layering is at or above the predetermined, threshold and at least the spatial change in luminance in the second image is at or above a predetermined threshold, multi-layering is decided to have occurred in the region of the relevant position.

Examples of indices for the spatial change in luminance include a variance of pixel values and/or a derivative sum of the pixel values relative to the spatial direction. This makes use of the fact that a site in a single-layered state has a small spatial change in luminance whereas a site that has become multi-layered has a greater spatial change in luminances. A region of a position at which the spatial change in luminance is appreciable is a site where multi-layering has occurred, or otherwise a portion of boundary between the interior and exterior of the cell aggregation; however, the degree of multi-layering as calculated by block matching is lower in the boundary portion, and is accordingly omitted in the above-described decision involving discerning the state of change toward becoming multi-layered.

Thus, using the degree of multi-layering of each of the parts of the cell aggregation at each of the points in time, the statistic based on the degree of similarity is calculated, and the degree of maturity of the cell aggregation is decided depending on the associated time lapse changes. As statistics based on the degree of similarity there can be cited (1) the sum of the degrees of multi-layering or (2) the occupancy of the multi-layered site.

### (1-1) Sum of degrees of multi-layering

In this technique, the degrees of multi-layering of each of the parts of the cell aggregation at each of the points in time as calculated in the manner described above (see FIG. 9B) for the entire cell aggregation (or, a designated region, in a case where an analysis range is designated) are added to determine the sum of the degrees of multi-layering at each of the points in time for each of the cell aggregations, and the time lapse changes of the sum of degrees of multi-layering are derived, whereby the state of maturity (degree of maturity) of the cell aggregation is decided on the basis of the resulting information on the time lapse changes.

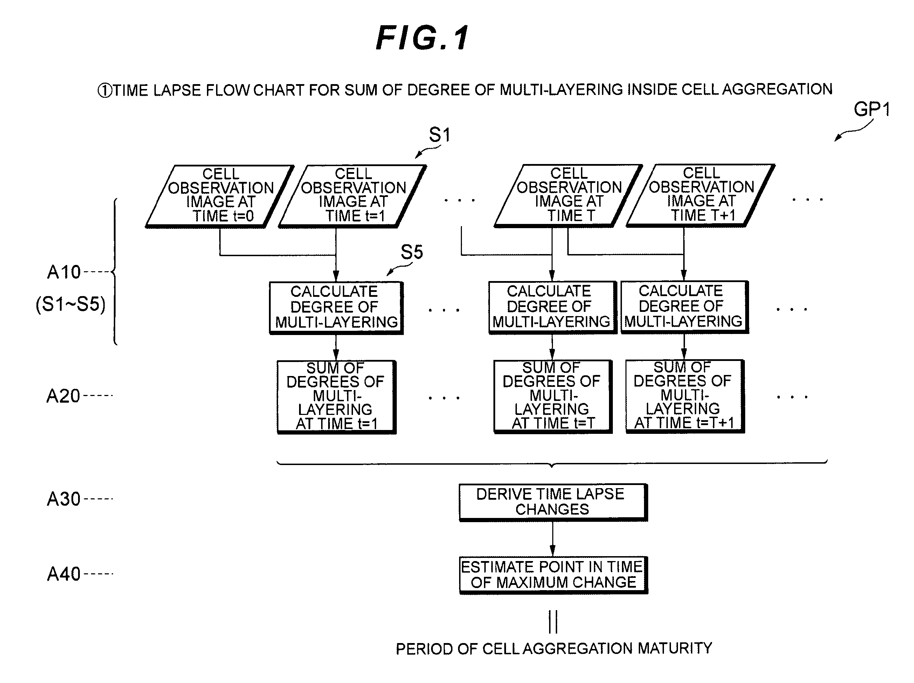

FIG. 1 illustrates a flow chart of the image processing program GP1 for determining the degree of maturity of a cell aggregation on the basis of the time lapse changes in the sum of the degrees of multi-layering, which includes the above-described program P1 for detecting a multi-layering site by block matching.

In the illustrated flow chart, step A10 includes the previously described program P1 for detecting a multi-layering site of a cell aggregation (S1 to S5); in step A10, the degree of multi-layering of each of the parts of the cell aggregation at time t+1 is calculated by block matching of the local region from the first image at time t and the second image at time t+1.

For example, the degree of multi-layering of each of the parts of the cell aggregation at time 1=1 is calculated from the cell observation image at time t=0 (the first image) and the cell observation image at time t=1 (the second linage) ; thereafter, block matching of the local region with the cell observation images at subsequent points in time is similarly performed in sequence, and the degree of multi-layering of each of the parts of the cell aggregation at time t=T+1 is calculated from the cell observation image at time t=T (the first image) and the cell observation image at time t=T+1 (the second image).

In step A20, the degrees of multi-layering of each of the parts of the cell aggregation at each of the points in time as calculated in step A10 are added for an entire cell aggregation, and the sums of the degrees of multi-layering at each of the points in time (time t=1, 2 , 3, ... , T, T+1, ...) are calculated for each of the cell aggregations. It is thereby possible to ascertain to what extent each of the cell aggregations has become multi-layered at each of the points in time.

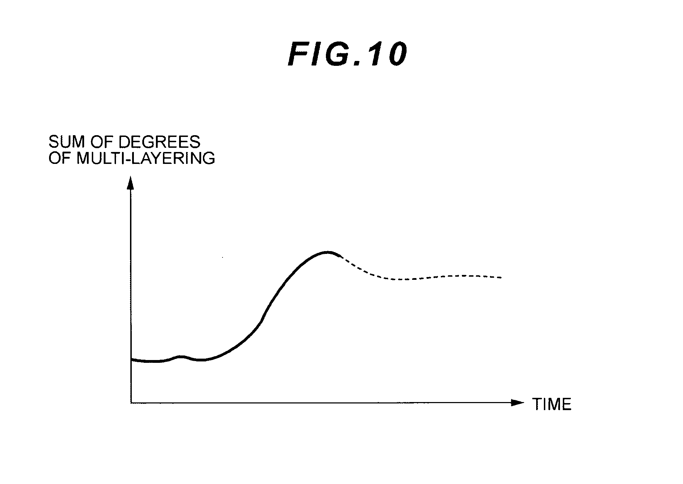

In step A30, the values of the sums of the degrees of multi-layering at each of the points in time as calculated in step A20 are lined up in time lapse along the points of time t=1, 2, 3, ..., T, T+1, ... , and the time lapse changes in the sums of the degrees of multi-layering are derived for each of the cell aggregations. FIG. 10 focuses on a single cell aggregation and illustrates an example of the output in a case where the temporal changes in the sums of the degrees of multi-layering calculated by the time lapse changes derivation processing in step A20 are displayed as a graph on the display panel 72. The horizontal axis in the drawing is the time elapsed from the time t=0, and the vertical axis is the sum of the degrees of multi-layering; the value of the sum is 0 to 1 in a case where the value is divided by the number of partitions of local regions and normalized.

In the initial stage of cell culturing, it is merely that the cells in the cell aggregation spread out in two dimensions, and since the structures of each of the individual cells are distinct, the sum of the degrees of multi-layering transitions by small values. When the cell aggregation grows and multi-layering begins, since the structures of the cells undergo intense three-dimensional changes and become more complex, the value of the sum of the degrees of multi-layering starts to increase and rises together with the expansion of the multi-layered region. When the multi-layered region spreads out to substantially the entire region of the cell aggregation, the rise of the value of the sum tails off, and essentially stops. Further, as time elapses, each of the individual cells in the region that has become multi-layered becomes very small at such a time and undergo fine structural changes; therefore, once the sum of the degrees of multi-layering reaches a maximum value, it transitions with a tendency to decrease slightly.

For this reason, the state of maturity of the cell aggregation can be decided from the information on the time lapse changes of the sum of the degrees of multi-layering (the "time lapse information on the degrees of multi-layering") as derived by the processing in step A30. For example, when the sum of the degrees of multi-layering first begins to rise in the time lapse information on the degrees of multi-layering, it is possible to decide that multi-layering growth has started in the relevant cell aggregation; and, when the sum of the degrees of multi-layering is rising, it is possible to decide that the cell aggregation is growing. When the sum of the degrees of multi-layering reaches a maximum value or exceeds a peak and enters a period of stability or a period of reduction, the cell aggregation can be decided to have matured.

The image processing program GP1 illustrated in FIG. 1 illustrates an example of a configuration in which there is a computation in step A40 as to whether or not the sum of the degrees of multi-layering has a maximum value from the time lapse information on the degrees of multi-layering as derived in step A30. A cell aggregation is decided to have matured in a case where the sum has a maximum value, the time thereof is calculated, and the determination results of the period of maturity of the cell aggregation are outputted.

The image analysis results can be displayed in an appropriate form on the display panel 72 or the like. In an aspect given by way of example, for individual cell aggregations, the transition of the sum of the degrees of multi-layering from t=0 until the current moment as illustrated in FIG. 10 is displayed in the form of a graph as time lapse information on the degrees of multi-layering; and, in a case where the cell aggregation is determined to have sufficiently matured, the time t of the period of maturity is displayed as the determination results. In a case where the observation images include a plurality of cell aggregations, an aspect is given by way of example wherein a cell aggregation that has been determined to have sufficiently matured is distinguished from a cell aggregation for which this is not the case, and color coding or another form of representation is used in the display. At such a time, a configuration may be adopted such that the color and/or luminance are altered and displayed in accordance with the degree of maturity of each of the cell aggregations (no growth yet, growth in progress, maturity preached).

### (1-2) The occupancy of the multi-layered site

According to this technique, for a site that has been identified as having become multi-layered on the basis of the degree of multi-layering of each of the parts of the cell aggregation, a calculation is made of the ratio by which the multi-layered site occupies the entire cell aggregation (or a designated region, in a case where an analysis range is designated) in the cell aggregation at each of the points in time; i.e., the occupancy of the multi-layered site is calculated, and the time lapse changes in the occupancy of the multi-layered site are derived, whereby the state of maturity (degree of maturity) of the cell aggregation is decided on the basis of the resulting information on the time lapse changes.

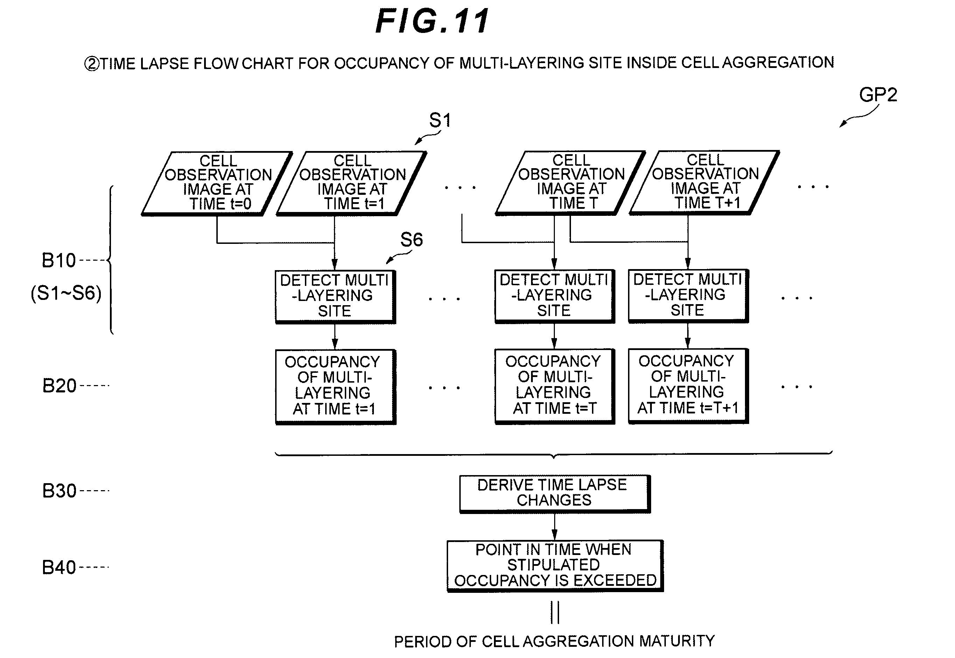

FIG. 11 illustrates a flow chart of the image processing program GP2 for determining the degree of maturity of a cell aggregation on the basis of the time lapse changes in the occupancy of a multi-layered site, which includes the previously described program P1 for detecting a multi-layering site by block matching.

In the illustrated flow chart, step B10 includes the previously described program P1 for detecting a multi-layering site of a cell aggregation (S1 to S6); in step B10, the degree of multi-layering of each of the parts of the cell aggregation at time t+1 is calculated by block matching of the local region from the first image at time t and the second image at time t+1, and a site that has become multi-layered is detected in accordance with the magnitude of the calculated degree of multi-layering.

For example, the multi-layered site of the cell aggregation at time t=1 is detected from the cell observation image at time t=0 (the first image) and the cell observation image at time t=1 (the second image), and block matching with the cell observation image at subsequent points in time is similarly performed in sequence; the multi-layered site of the cell aggregation at time t=T+1 is calculated from the cell observation image at time t=T (the first image) and the cell observation image at time t=T+1 (the second image).

In step B20, the occupancy (surface area ratio) of the multi-layered sites accounting for each of the cell aggregations is calculated for the multi-layered sites detected in step B10, and the occupancy for the multi-layered sites at each of the points in time (time t=1, 2, 3, ..., T, T+1, ...) is calculated for each of the cell aggregations. It is thereby possible to ascertain to what extent each of the cell aggregations has become multi-layered at each of the points in time.

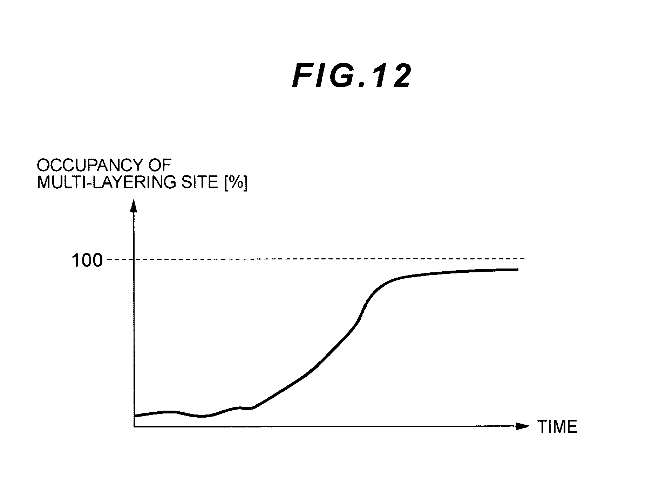

In step B30, the values of the occupancy of the multi-layered site at each of the points in time as calculated in step B20 are lined up in time lapse along the points of time t=1, 2, 3, ..., T, T+1, ...., and the time lapse changes in the occupancy of the multi-layered site are derived for each of the cell aggregations. FIG. 12 illustrates an example of the output for a single cell aggregation in a case where the temporal changes in the occupancy of the multi-layered site calculated by the time lapse changes derivation processing in step B20 are displayed in the form of a graph on the display panel 72. The horizontal axis in the drawing is the time elapsed from the time t=0, and the vertical axis is the occupancy of the multi-layered site, which is a value of 0% to 100%.

In the initial stage of cell culturing, there exist almost no multi-layered sites; therefore, the occupancy of multi-layered sites transitions at a small value. When the cell aggregation grows and multi-layering begins, the occupancy begins to increase and rises together with the expansion of the multi-layered region. When the multi-layered region spreads out to substantially the entire region of the cell aggregation, the rise in occupancy tails off until there is substantially no rise in the occupancy.

Accordingly, the maturity state of the cell aggregation can be decided from the time lapse change information on the occupancy of the multi-layered site (the "time lapse information on the multi-layered occupancy") as derived by the processing in step B30. For example, when the occupancy of the multi-layered site first begins to rise in the time lapse information on the multi-layering occupancy, it is possible to decide that the multi-layering growth on the cell aggregation has begun; and, when occupancy of the multi-layered site is rising, to decide that growth is in progress. When the occupancy of the multi-layered site is at or above a predetermined value, it is possible to decide that the cell aggregation has matured.

The image processing program GP2 illustrated in FIG. 11 illustrates an example of a configuration in which there is a comparison in step B40 between the occupancy of the multi-layered site at each of the points of time and a stipulated occupancy that is pre-set as a standard for determining the degree of maturity, from the time lapse information on the multi-layered occupancy derived in step B30. A cell aggregation is decided to have matured in a case where the occupancy of the multi-layered site of the cell aggregation is at or above the stipulated occupancy. The time at which the stipulated occupancy is exceeded is calculated, and the determination results of the period of maturity of the cell aggregation are outputted. The stipulated occupancy is set appropriately in accordance with the type and/or properties of the cells to be observed, the purpose of observation, or other parameter, but, in the case of iPS cells, ES cells, or other cells, is generally set in the range of about 70% to 90%.

The image analysis results can be displayed in an appropriate form on the display panel 72 or the like. An example is illustrated in an aspect where, as time lapse information on the multi-layering occupancy, the transition in occupancy from t=0 until the current moment is displayed in the form of a graph together with an index line of the stipulated occupancy (for example, 80%) on the graph illustrated in FIG. 12 for each of the individual cell aggregations, and the determination results of the degree of maturity of the cell aggregations are displayed. In a case where the cell aggregations are decided to have matured, the time t at which the stipulated occupancy is exceeded is also displayed. In a case where the observation images include a plurality of cell aggregations, examples include an aspect in which a distinction is made between cell aggregations that have been determined to have sufficiently matured and cell aggregations for which this is not the case, and a display is presented using color coding or another display, or an aspect in which the color and/or luminance are altered and displayed in accordance with the degree of maturity of each of the cell aggregationes (no growth yet, growth in progress, maturity reached

Thus, according to the above-described technique (I) for using the degree of similarity by block matching of local regions between images, it is possible to quantitatively decide the degree of maturity of a cell aggregation from information on time lapse changes derived by calculating a multi-layering feature value (the sum of the degrees of multi-layering, or the occupancy of a multi-layered site) from two images over a predetermined time interval, based on the degree of similarity of a local region between the images, and then deriving the time lapse changes thereof. There follows a description of a technique for determining the degree of maturity using a "statistic based on the pixel values in the neighborhood of the contours of the cell aggregation" as multi-layering feature values.

### (II) Technique for using a statistic based on the pixel values in the neighborhood of the contours of the cell aggregation

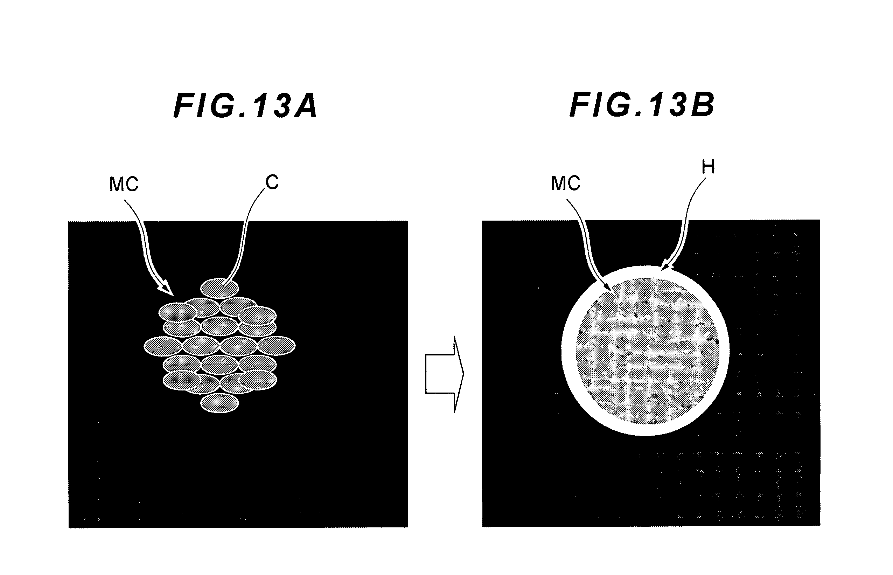

FIG. 13A-B provides a schematic illustration of an observation image of when cells have been cultured in a flat manner. In the initial stage of culturing, as illustrated in FIG. 13A, the aggregated cells C adhere to the Petri dish or other base of the cell culture medium, and the cell aggregation spreads out in two dimensions. Therefore, very little of a so-called "halo" occurs in the neighborhood of the contours of the cell aggregation MC. On the other hand, when the cell aggregation grows and becomes multi-layered, proliferating in three dimensions, then a thickness appears at the contour parts of the cell aggregation; therefore, a halo H occurs at the neighborhood of the contours of the cell aggregation MC, as illustrated in FIG. 13B. The halo of the neighborhood of the contour parts appears more clearly in a case where the observation optical system (54a, 55a) is a phase-contrast microscope.

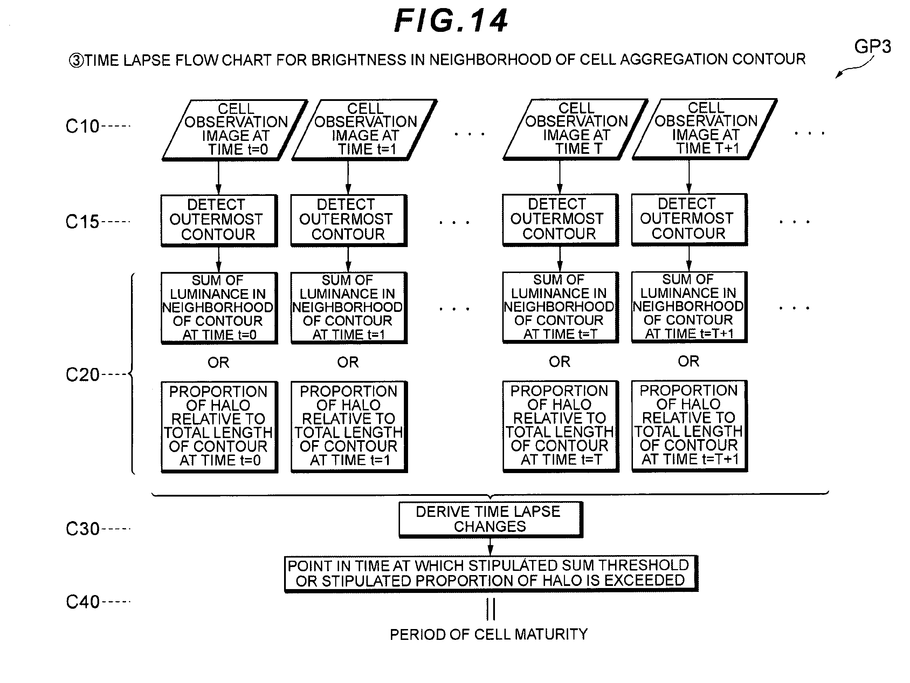

This technique makes use of the property whereby, in concert with the multi-layering and maturation of the cell aggregation as described above, a halo is generated in the neighborhood of the contours and the pixel values change. Examples of statistics based on the pixel values include the sum of luminances, in which the pixel values in the neighborhood of the contours of the cell aggregation are summed up, as well as the proportion of the length, along the contours, of a portion having a certain pixel value or higher (the halo), relative to the overall length of the contours of the cell aggregation (halo length/overall length of the contours) An image processing program GP3, which has been set and stored in the ROM 62, is read into the CPU 61, and the determination of the degree of maturity of the cell aggregation using such statistics is achieved by the sequential execution, by the CPU 61, of processing that is based on the image processing program GP3. FIG. 14 illustrates a flow chart of the image processing program GP3.

First, in step C10, the image analysis unit 120 reads out time lapse images from times t=0, 1, 2, 3, ..., T, T+1, ..., which have been recorded in the RAM 63; in step C15, the outermost contour of the cell aggregation is extracted and segmented for the observation images at each of the points in time. The Snakes method, the Level Set method, or another dynamic contour extract method is used to extract the outermost contour. At such a time, in a case where the observation images include a plurality of segmented cell aggregations, each of the cell aggregations MC is labeled and a correspondence is made for the cell aggregations between images, as illustrated in PIG. 7.

In step C20, a statistic described above based on the pixel values is calculated for the contour parts of the cell aggregation at each of the points of time as extracted in step C15. For example, in a case where the statistic that is based on the pixel value is the sum of luminances in the neighborhood of the contours, the sum of the pixel values is calculated for the adjacent region along the contours of the cell aggregation. The size of the adjacent region, i.e., the width of the frame in the peripheral direction relative to the contour line extracted in step C15, is appropriately set in accordance with the region in which the halo appears as observed with the relevant observation system when the cell aggregation to be observed has become multi-layered. In a case where the statistic that is based on the pixel values is the proportion of the length of the halo relative to the overall length of the contours of the cell aggregation, then the proportion of the length, along the contour line, of the portion having a certain pixel value or higher in the adjacent region of the cell aggregation is calculated relative to the overall length of the contours of the contour line of the cell aggregation as extracted in step C15.

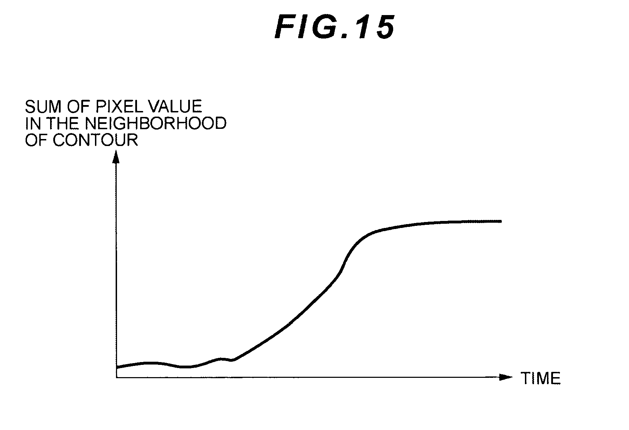

In step C30, the statistics that are based on the pixel values at each of the points of time, as calculated in step C20, are lined up in time lapse along the points of time t=1, 2, 3, ..., T, T+1, ..., and the time lapse changes in the statistics that are based on the pixel values are derived for each of the cell aggregations. FIG. 15 illustrates an example of the output for a single cell aggregation in a case where the temporal changes in the sum of luminances in the neighborhood of the contours as calculated by the time lapse changes derivation processing in step C20 are displayed in the form of a graph on the display panel 72. The horizontal axis in the drawing is the time elapsed from the time t=0, and the vertical axis is the sum of the pixel values in the neighborhood of the contours.

As is schematically illustrated in FIG. 13A, in the initial stage of cell culturing, the aggregated cells C adhere to the base of the cell culture medium, and the cell aggregation spreads out in two dimensions; therefore, almost no halo appears on each of the individual cells C or in the neighborhood of the contours of the aggregated cell aggregation MC, and the state of change in the sum of the luminances in the neighborhood of the contours is low. When the cell aggregation grows and multi-layering begins, a bright halo appears in the neighborhood of the contours near the site at which the thickness has become larger due to multi-layering, and the sum of the luminances begins to increase gradually, becoming larger as the multi-layered region expands. Then, as in FIG. 13B, when the multi-layered region spreads out to substantially the entire region of the cell aggregation MC, a bright halo surrounds the entirety of the cell aggregation and the increase in the sum of the luminance in the neighborhood of the contours substantially stops. The time lapse changes also have a similar transition in a case where the proportion of the length of the halo relative to the overall length of the contours of the cell aggregation is used as the statistic that is based on the pixel values.

Accordingly, the state of maturity of the cell aggregation can be decided from the information on the time lapse changes in the statistic that is based on the pixel values as derived by the processing in step C30 (the "time lapse information on the luminance statistic"). For example, when the sum of the luminances in the neighborhood of the contours first begins to increase in the time lapse information on the luminance statistic, it can be decided that multi-layering growth has started in the relevant cell aggregation, and when the sum of the luminances is becoming larger, it can be decided that growth is in progress. It is then possible to decide that the relevant cell aggregation has matured when the tendency of the sum of the luminances to increase becomes more gradual and the rate of increase is at a predetermined value or below, or when the sum of the luminances is at a stipulated sum threshold or above.

In the image processing program GP3 illustrated in FIG. 14, there is a comparison in step C40 between the sum of the luminances in the neighborhood of the contours at each of the points of time and a stipulated sum threshold that is pre-set as a standard for determining the degree of maturity, from the time lapse information on the luminance statistic derived in step C30. A cell aggregation is decided to have matured in a case where the sum of the luminances in the neighborhood of the contours is at or above the stipulated sum threshold, the time at which the stipulated sum threshold is exceeded is calculated, and the determination results of the period of maturity of the cell aggregation are outputted. Similarly, in a case where the statistic that is based on the pixel values is the proportion of the length of the halo relative to the overall length of the contours of the cell aggregation, there is a comparison between the proportion of the length of the halo relative to the overall length of the contours of the cell aggregation at each of the points in time and a stipulated halo proportion that is pre-set as a standard for determining the degree of maturity. A cell aggregation is decided to have matured in a case where the proportion of the length of the halo relative to the overall length of the contours is at or above the stipulated proportion, and the determination results are outputted taking the time at which the stipulated proportion is exceeded as the maturity period. The image analysis results can be displayed on the display panel 72 or the like as appropriate, similarly with respect to each of the previously described structural modes.

As has been described above, according to the technique (II) for using the statistic that is based on the pixel values in the neighborhood of the contours of the cell aggregation, it is possible to quantitatively decide the state of maturity of a cell aggregation from information on time lapse changes derived by calculating multilayer feature values (the sum of the luminances in the neighborhood of the contours, or the proportion of the length of the halo relative to the overall length of the contours) based on the pixel values in the neighborhood of the contours of the cell aggregation at each of the points in time from a group of time lapse images separated by a predetermined time interval, and then deriving the time lapse changes thereof. The following is a description of a technique for determining the degree of maturity using a "statistic that is based on the contour shape of the cell aggregation" as the multi-layering feature value.

### (III) Technique for using a statistic that is based on the contour shape of the cell aggregation

As illustrated in FIG. 13A, in the initial stage of culturing, because the cells C aggregate and a cell aggregation MC forms, individual cells are present in the neighborhood of the contours of the cell aggregation MC, which spreads out in two dimensions, and the contour shape of the cell aggregation takes on a complicated shape that includes many concavities and convexities. As the cell aggregation progressively grows, the concavities and convexities of the contour parts are gradually assimilated, and the contour shape becomes smoother; by the time the cells form a three-dimensional structure due to multi-layering, the contour shape of the cell aggregation MC will have become relatively more rounded, as illustrated in FIG. 13B.

This technique makes use of the property whereby the contour shape of the cell aggregation changes in this manner during the stage of the cell aggregation maturing. The degree of complexity of the contours of the cell aggregation is given as a representative example of a statistic that is based on the contour shape of the cell aggregation. The degree of complexity of the contours of the cell aggregation can be stipulated by the proportion of the perimeter length relative to the surface area of the cell aggregation (perimeter / surface arena).

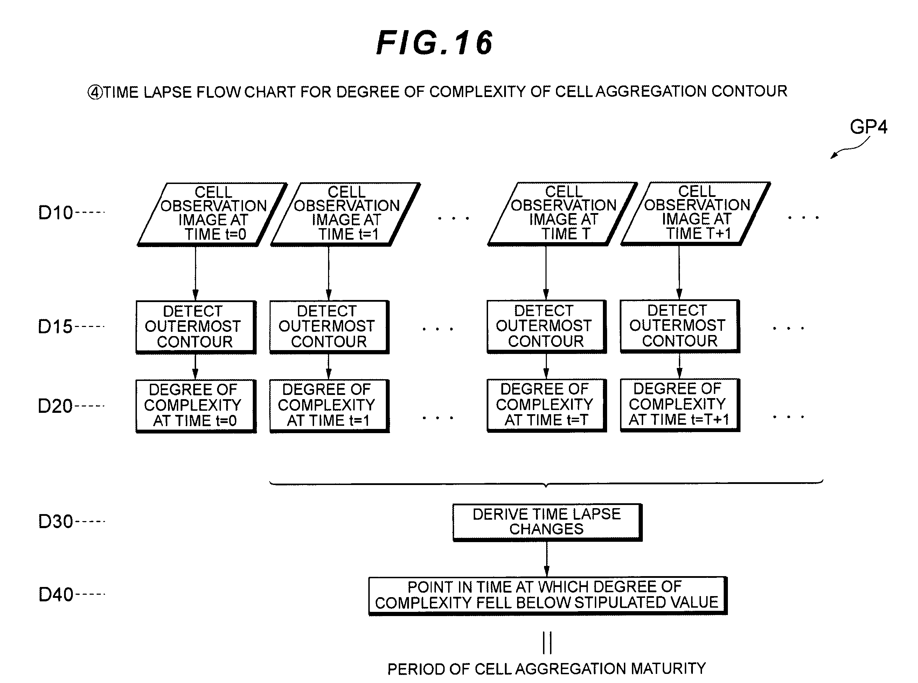

An image processing program GP4 is read into the CPU 61, and the determination of the degree of maturity of the cell aggregation is achieved by the sequential execution, by the CPU of processing that is based on the image processing program GP4. FIG. 16 illustrates a flow chart of the image processing program GP4.

In step D10, the image analysis unit 120 reads out time lapse images from times t=0, 1, 2, 3, ..., T, T+1, ..., and in step D15, the outermost contour is extracted and segmented for the observation images at each of the points in time, by a dynamic contour extraction method. In a case where the observation images include a plurality of segmented cell aggregations, each of the cell aggregations is labeled and a correspondence is made for the cell aggregations between images (FIG. 7) .

In step D20, a statistic described above based on the contour shape is calculated for the contour parts of the cell aggregation at each of the points of time as extracted in step D15. In this embodiment, the proportion of the perimeter length (the overall length of the contours) relative to the surface area of the cell aggregation, the outermost contours of which have been extracted, is calculated for the degree of complexity of the contours of the cell aggregation.

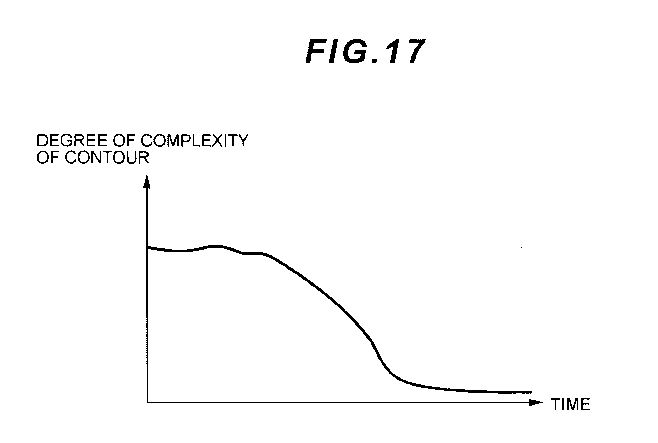

In step D30, the statistics that are based on the contour shape at each of the points of time, as calculated in step D20, are lined up in time lapse along the points of time t=1, 2, 3, ..., T, T+1, ..., and the time lapse changes in the statistics that are based on the contour shape are derived for each of the cell aggregations. FIG. 17 illustrates an example of the output for a single cell aggregation in a case where the temporal changes in the degree of complexity of the contours of the cell aggregation as calculated by the time lapse changes derivation processing in step D20 are displayed in the form of a graph on the display panel 72. The horizontal axis in the drawing is the time elapsed from time 1=0, and the vertical axis is the degree of complexity of the contours.

As illustrated in FIGS. 13A and 13B, in the initial stage of cell culturing, the cells aggregate, the cell aggregation spreads out in two dimensions, and the contours of the cell aggregation assume a complex shape including many concavities and convexities; therefore, the degree of complexity of the contours as calculated in step D20 shifts at a high value. As the cell aggregation progressively grows, the concavities and convexities of the contour parts are gradually assimilated, and the contour shape becomes smoother; the degree of complexity of the contours decreases as time elapses. Furthermore, as multi-layering progresses inside the cell aggregation, the contour of the cell aggregation becomes ellipsoidal or circular, and the degree of complexity of the contours is a small value that essentially stops decreasing.

Therefore, the state of maturity of the cell aggregation can be decided from the information on the time lapse changes in the statistic that is based on the contour shape of the cell aggregation as derived by the processing in step D30 (the "time lapse information on the contour shape statistics"). For example, when the degree of complexity of the contours first begins to decrease in the time lapse information on the contour shape statistic, it is possible to decide that the relevant cell aggregation is in a period of transitioning towards becoming multi-layered; and, when the degree of complexity of the contours is decreasing, it is possible to decide that multi-layering growth is in progress. Then, when the tendency of the degree of complexity of the contours to decrease becomes more gradual and the rate of decrease is at a predetermined value or below, or when the degree of complexity of the contours is at a stipulated degree of complexity or below, then it is possible to decide that the relevant cell aggregation has matured.

In the image processing program GP4 illustrated in FIG. 16, there is a comparison in step D40 between the degree of complexity of the contours of the cell aggregation at each of the points of time and a stipulated degree of complexity that is pre-set as a standard for determining the degree of maturity, from the time lapse information on the contour shape statistic derived in step D30. A cell aggregation is decided to have matured in a case where the degree of complexity of the contours is at or below the stipulated degree of complexity, the time at which the degree of complexity falls below the stipulated value is calculated, and the determination results of the period of maturity of the cell aggregation are outputted. The image analysis results can be displayed on display panel 72 or the like as appropriate, similarly with respect to each of the previously described forms of configuration.

Therefore, according to the technique (III) described above for using the statistic that is based on the contour shape of the cell aggregation, it is possible to quantitatively decide the state of maturity of a cell aggregation from information on time lapse changes derived by calculating multi-layering feature values (the degree of complexity of the contours of the cell aggregation) based on the contour shape of the cell aggregation at each of the points in time from a group of time lapse images separated by a predetermined time interval, and then deriving the time lapse changes thereof.

As has been described above, the image processing device 100 of the present invention sequentially calculates feature values relating to the multi-layering of the cell aggregation from a group of time lapse images in which images of a cell aggregation are taken by an imaging device over a predetermined time interval, derives the time lapse changes, and decides the degree of maturity of the cell aggregation on the basis of the time lapse changes of the feature values relating to the multi-layering. Therefore, according to the present invention, there can be provided means by which the degree of maturity of a cell aggregation can be decided from time lapse images taken by an imaging device without the cells being damaged by the administration of a reagent.

The embodiments described above illustrate examples of a configuration in which the cell culture observation system BS reads out time lapse images (image data) that are taken by an imaging device and stored in the RAM 63 and analyzes the multi-layering state, but the configuration may be such that the images taken by the imaging device are analyzed in real-time as sequential first and second images and the degree of maturity of the cell aggregation at the current point in time is displayed. The configuration may also be such that time lapse images taken in another observation system and recorded in a magnetic storage medium, or the like, time lapse images that have been forwarded via a communication line, or the like are read in and the state of maturity is analyzed. The configuration may further be such that an operator uses a mouse or the like to set a predetermined range of the observation images (for example, a specific cell aggregation, or a specific site in a cell aggregation) as an analysis range, and the image processing device executes the analysis of the state of maturity for the set analysis range.

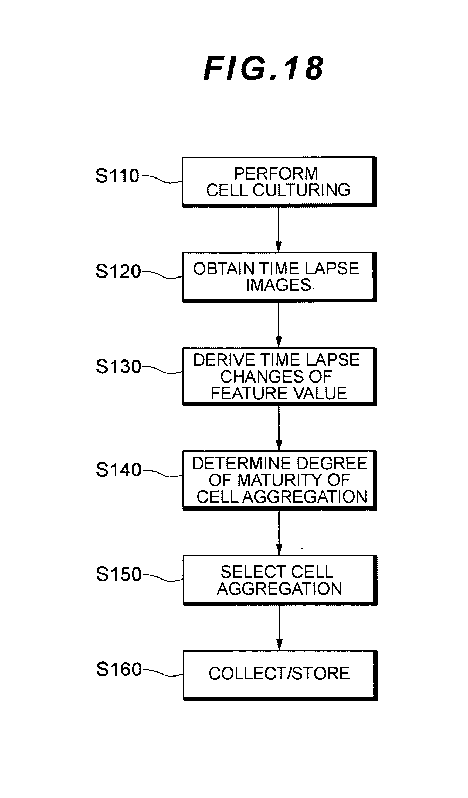

The following is a description of the method for producing a cell aggregation according to an embodiment of the present invention, with reference to FIG. 18. Specifically, the method for producing a cell aggregation comprises a cell culture step for culturing cells (S110) and determination steps for observing, using the above-described image processing device, the cells cultured in the cell culture step and then determining the degree of maturity of a cell aggregation in the cells, which vary by cell culture (S120 to S140).

More specifically, the method for producing a cell aggregation is configured to comprise a cell culture step for culturing cells (S110), an obtainment step for taking, by an imaging device over a predetermined time interval, images of the cells cultured in the cell culture step and for obtaining time lapse images of a cell aggregation in the cells, which vary by cell culture (S120); a derivation step for sequentially calculating feature values relating to the multi-layering of the cell aggregation, from the time lapse images obtained in the obtainment step, and deriving the time lapse changes of the calculated feature values relating to the multi-layering (S130) ; a determination step for determining the degree of maturity of a cell aggregation on the basis of the time lapse changes of the feature values relating to the multi-layering as derived in the derivation step (S140); a selection step for selecting a cell aggregation on the basis of a predetermined standard (S150) ; and a collection and storage step for collecting and storing the selected cell aggregation (S160). The cells that are cultured may be human-derived cells; cells derived from cows, horses, pigs, mice, or other animals; or plant-derived cells. The cell aggregation may be stored using cryogenic storage.

### EXPLANATION OF NUMERALS AND CHARACTER

- A:: Local region
- BS:: Cell culture observation system
- C:: Cell
- MC:: Cell aggregation
- P1, GP1 to GP4:: Image processing programs
- 5:: Observation unit
- 6:: Control unit
- 54:: Macro observation system
- 54c:: Imaging device
- 55:: Microscope observation system
- 55c:: Imaging device
- 61:: CPU (computer)
- 62:: ROM
- 63:: RAM
- 100:: Image processing device
- 120:: Image analysis unit
- 130:: Output unit

## Claims

1. A technique for determining the maturity of a cell aggregation, comprising the steps of:
obtaining time lapse images in which images of a cell aggregation are taken by an imaging device over a predetermined time interval;
sequentially calculating a feature value relating to multi-layering of the cell aggregation from the obtained time lapse images, and deriving time lapse changes of the calculated feature value relating to the multi-layering; and
determining the degree of maturity of the cell aggregation on the basis of the time lapse changes of the feature value relating to the multi-layering.

2. The technique for determining the maturity of a cell aggregation according to Claim 1, **characterized in that**:
the feature value relating to the multi-layering is a statistic based on the degree of similarity of a region at a position with the highest degree of matching calculated for a first image at a time t and a second image at a time t+1 in the time lapse images, using the luminance distribution of a local region of the cell aggregation in the first image as a template and performing block matching for the luminance distribution of a nearby part including a corresponding position of the cell aggregation in the second image.

3. The technique for determining the maturity of a cell aggregation according to Claim 1, **characterized in that**:
the feature value relating to the multi-layering is a statistic that is based on a pixel value in the neighborhood of a contour of the cell aggregation.

4. The technique for determining the maturity of a cell aggregation according to Claim 1, **characterized in that**:
the feature value relating to the multi-layering is a statistic that is based on a shape of the contour of the cell aggregation.

5. An image processing program that can be read out by a computer, the image processing program being adapted for causing the computer to function as an image processing device for obtaining an image taken by an imaging device and performing image processing, comprising the steps of:
obtaining time lapse images in which images of a cell aggregation are taken by an imaging device over a predetermined time interval;
sequentially calculating a feature value relating to multi-layering of the cell aggregation from the obtained time lapse images;
deriving time lapse changes of the calculated feature value relating to the multi-layering; and
outputting information on the derived time lapse changes of the feature value relating to the multi-layering.

6. The image processing program according to Claim 5, **characterized in that**:
the feature value relating to the multi-layering is a statistic based on the degree of similarity of a region at a position with the highest degree of matching calculated for a first image at a time t and a second image at a time t+1 in the time lapse images, using the luminance distribution of a local region of the cell aggregation in the first image as a template and performing block matching for the luminance distribution of a nearby part including a corresponding position of the cell aggregation in the second image.

7. The image processing program according to Claim 5, **characterized in that**:
the feature value relating to the multi-layering is a statistic that is based on a pixel value in the neighborhood of a contour of the cell aggregation.

8. The image processing program according to Claim 5, **characterized in that**:
the feature value relating to the multi-layering is a statistic that is based on a shape of the contour of the cell aggregation.

9. The image processing program according to any of Claims 5 to 8, **characterized by** comprising the steps of:
determining the degree of maturity of the cell aggregation on the basis of the information on the time lapse changes of the feature value relating to the multi-layering; and
outputting a result of the determination.

10. The image processing program according to Claim 9, **characterized in that**:
in a case where the time lapse images include a plurality of cell aggregations,
the step for outputting the degree of maturity information is configured such that the degree of maturity is determined for each of the cell aggregations, a distinction is made between matured cell aggregations and immature cell aggregations, and a result of the distinction is outputted.

11. An image processing device provided with an image analysis unit for obtaining time lapse images in which images of a cell aggregation are taken by an imaging device over a predetermined time interval and analyzing the images, and an output unit for outputting a result of the analysis performed by the image analysis unit,
the image processing device being configured such that:
the image analysis unit sequentially calculates a feature value relating to multi-layering of the cell aggregation from the time lapse images, and derives time lapse changes in the calculated feature value relating to the multi-layering; and
the output unit outputs information on the time lapse changes of the feature value relating to the multi-layering as derived by the image analysis unit.

12. The image processing device according to Claim 11, **characterized in that**:
the feature value relating to the multi-layering is a statistic based on the degree of similarity of a region at a position with the highest degree of matching calculated for a first image at a time t and a second image at a time t+1 in the time lapse images, using the luminance distribution of a local region of the cell aggregation in the first image as a template and performing block matching for the luminance distribution of a nearby part including a corresponding position of the cell aggregation in the second image.

13. The image processing device according to Claim 11, **characterized in that**:
the feature value relating to the multi-layering is a statistic that is based on a pixel value in the neighborhood of a contour of the cell aggregation.

14. The image processing device according to Claim 11, **characterized in that**:
the feature value relating to the multi-layering is a statistic that is based on a shape of the contour of the cell aggregation.

15. The image processing device according to any of Claims 11 to 14, **characterized in that**:
the image analysis unit determines the degree of maturity of the cell aggregation on the basis of the information on the time lapse changes of the feature value relating to the multi-layering; and
the output unit outputs a result of the determination as determined by the image analysis unit.

16. The image processing device according to Claim 15, **characterized in that**:
in a case where the time lapse images include a plurality of cell aggregations,
the image analysis unit determines the degree of maturity for each of the cell aggregations, and distinguishes between a matured cell aggregation and an immature cell aggregation; and
the output unit outputs a result of the distinguishing as distinguished by the image analysis unit.

17. A method for producing a cell aggregation, comprising:
a cell culture step for culturing cells; and
an identification step for observing, using the image processing device according to any of Claims 11 to 16, the cells cultured in the cell culture step, and identifying the degree of maturity of a cell aggregation in the cells, which vary by cell culture.

18. A method for producing a cell aggregation, comprising:
a cell culture step for culturing cells:
an obtainment step for taking images of the cells cultured in the cell culture step by an imaging device over a predetermined time interval and for obtaining time lapse images of a cell aggregation in the cells, which vary by cell culture;
a derivation step for sequentially calculating a feature value relating to multi-layering of the cell aggregation, from the time lapse images obtained in the obtainment step, and deriving time lapse changes of the calculated feature value relating to the multi-layering; and
a determination step for determining the degree of maturity of a cell aggregation on the basis of the time lapse changes of the feature value relating to the multi-layering as derived in the derivation step.
